# EUROPÄISCHE PATENTANMELDUNG

(11) **EP 2 163 621 A1**
(43) Veröffentlichungstag der Anmeldung: **17.03.2010**
(21) Anmeldenummer: 08163624.3
(22) Anmeldetag: 03.09.2008
(51) Int. Cl.: C12N 15/10

(54) **Verfahren zum Isolieren und Reinigen von Nukleinsäuren**

(71) Anmelder: QIAGEN GmbH, 40724 Hilden (DE)
(72) Erfinder: Himmelreich, Ralf, 40764 Langenfeld (DE); Werner, Sabine, 40227 Düsseldorf (DE)
(74) Vertreter: Vossius & Partner

(57) **Zusammenfassung**

Die vorliegende Erfindung betrifft ein Verfahren zum Isolieren und Reinigen von Nukleinsäuren durch Elution der Nukleinsäuren von Nukleinsäurehaltigen Proben, sowie biologischen Materialien. Die vorliegende Erfindung betrifft weiterhin ein Kit zur Durchführung des erfindungsgemäßen Verfahrens.

## Beschreibung

Die vorliegende Erfindung betrifft ein Verfahren zum Isolieren und Reinigen von Nukleinsäuren durch Separation der Nukleinsäuren von Nukleinsäurehaltigen Proben, sowie biologischen Materialien. Die vorliegende Erfindung betrifft weiterhin ein Kit zur Durchführung des erfindungsgemäßen Verfahrens.

Ein Verfahren zum Isolieren und Reinigen von Nukleinsäuren, welches im Stand der Technik bereits bekannt ist, basiert auf der Adsorption von Nukleinsäuren auf Glas- oder Silicapartikeln in Anwesenheit von chaotropen Salzen, gefolgt von der Wiedergewinnung der adsorbierten Nukleinsäuren (Vogelstein, B. und Gillespie, D. (1979); "Preparative and analytical purification of DNA from Agarose", Proc. Natl. Acad. Sci. USA 76: 615-619). Gemäß diesem Verfahren wird unter Verwendung von hohen Konzentrationen an chaotropen Salzen, wie beispielsweise Natriumiodid, Natriumperchlorat oder Guanidiniumthiocyanat, DNA isoliert und über Agarose gereinigt. Die RNA oder DNA kann auch aus verschiedenen Mischungen isoliert oder gereinigt werden (Boom, R., 1990; "Rapid and simple method for purification of nucleic acids", J. Clin. Microbiol. 28: 495-503).

Nach erfolgter Nukleinsäurereinigung werden Nukleinsäuren häufig in der Polymerasekettenreaktion (PCR) verwendet. Die PCR amplifiziert Nukleinsäuren in einer Sequenz-spezifischen Weise und wird deshalb breit in der Gen- oder DNA-Diagnose angewendet. Die Anwendung der PCR-Technik in klinischen Routineverfahren bringt mehrere Probleme mit sich. Es ist bekannt, dass inhibitorische Substanzen, die nicht aus dem Nukleinsäurereinigungsschritt entfernt worden sind, die PCR inhibieren können. Solche inhibitorischen Substanzen sind beispielsweise Hämoglobin und Tenside, die im Nukleinsäureextraktionsprozess verwendet wurden. Aus diesem Hintergrund wird ersichtlich, dass die Verfahren zum Extrahieren und Reinigen von Nukleinsäuren sehr wichtig und relevant sind (Oshima et al., JJCL A, 22(2) 145-150 (1997)).

Verfahren zur Extraktion und Reinigung von Nukleinsäuren werden sehr häufig automatisiert. Es gibt bereits im Stand der Technik automatisierte Nukleinsäureextraktionsverfahren, solche sind beschrieben in JP-A-107854/1999 und in JP-A-266864/1999. In den meisten Verfahren zur Isolation und Reinigung von Nukleinsäuren wird eine Lösung, die in hoher Konzentration Salze enthält und in hoher Konzentration Alkohol enthält, und in welcher sich die Nukleinsäuren befinden, in Kontakt mit einer Adsorptionsoberfläche gebracht. Die Adsorptionsoberfläche kann in Form eines Harzes in einer Säule vorliegen. Dann werden die Nukleinsäuren an diese Oberfläche adsorbiert und später mit Hilfe von Lösungen eluiert, die weniger hoch konzentrierte Salzlösungen enthalten.

Das Problem der meisten Verfahren zur Isolation und Reinigung von Nukleinsäuren ist, dass die Ausbeute der Nukleinsäuren relativ gering ist. Ein weiteres Problem ist, dass Ethanol-haltige Lösungen gemäß den Regularien der IATA (International Air Transport Association) als gefährliche Materialien eingestuft werden (HAZMAT; Hazardous Materials). Gemäß den IATA Regularien werden alle Produkte, Materialien und Güter in neun Hauptklassen klassifiziert. Durch die Einstufung als gefährliche Güter werden beim Transport durch Flugzeuge zusätzliche Gebühren und Steuern fällig. Aufgabe der vorliegende n Erfindung war es daher, Ethanol (oder Isopropanol) im Verfahren zur Reinigung und Extraktion von Nukleinsäuren weitgehend zu ersetzen, um die Isolation und Reinigung von Nukleinsäuren zu erleichtern, ein Ethanolfreies Verfahren bereitzustellen und den Luftfrachttransport zu erleichtern.

Aus dem Stand der Technik sind Substitute für Alkohol in Verfahren zur Reinigung von Nukleinsäuren bekannt, die jedoch die oben besprochen Probleme nur teilweise lösen (US 2004/0167324). Die Mehrheit der hierin beschriebenen Substanzen fallen entweder in die HAZMAT IATA Regularien oder weisen einen stechenden Geruch auf, der eine Präparation nur im Abzug erlaubt.

Überraschenderweise wurden im Rahmen der vorliegenden Erfindung Lösungsmittel für Waschpuffer zum Waschen von an Oberflächen immobilisierten Nukleinsäuren gefunden, die es erlauben, dass der Waschpuffer im Wesentlichen frei von Alkohol, das heißt Ethanol ist.

Gegenstand der vorliegenden Erfindung ist somit ein Verfahren zum Waschen von an Oberflächen immobilisierten Nukleinsäuren, wobei an Oberflächen immobilisierte Nukleinsäuren in Kontakt mit einem Waschpuffer gebracht werden und der Waschpuffer im Wesentlichen frei von Ethanol ist.

Im Wesentlichen frei von Ethanol bedeutet im Rahmen der vorliegenden Erfindung, dass Ethanol in einer Konzentration von weniger als 24 Vol%, bevorzugt von weniger als 16 Vol% und am meisten bevorzugt gar nicht im Waschpuffer vorliegt.

Gegenstand der vorliegenden Erfindung ist insbesondere ein Verfahren zum Waschen von an Oberflächen immobilisierten Nukleinsäuren, wobei der Waschpuffer Lösungsmittel enthält welche ausgewählt sind aus der Gruppe enthaltend C3- bis C5 Alkyldiole, sowie kurzkettige Ethylengylcol-Derivate und diverse wasserlösliche polymere Verbindungen, wobei der Waschpuffer im wesentlichen frei von Ethanol ist.

In einer bevorzugten Ausführungsform ist Gegenstand der vorliegenden Erfindung ein Verfahren zum Waschen von an Oberflächen immobilisierten Nukleinsäuren, wobei der Waschpuffer Lösungsmittel enthält welche ausgewählt sind aus der Gruppe enthaltend 1,2-Butandiol, 1,2-Propandiol, 1,3-Butandiol, 1-Methoxy-2-propanolacetat, 3-Methyl-1,3,5-pentantriol, DBE-2 dibasische Ester, DBE-3 dibasische Ester, DBE-4 dibasische Ester, DBE-5 dibasische Ester, DBE-6 Dimethyladipat, Diethylenglycolmonoethylether (DGME), Diethylenglycolmonoethyletheracetat (DGMEA), Ethyllactat, Ethylenglycol, Poly(2-ethyl-2-oxazolin), Poly(4-styrolsulfonsäure-co-maleinsäure)-Natrium-Salzlösung, Tetraethylenglycol (TEG), Tetraglycol (Tetrahydrofurfurylpolyethylenglycolether), Tetrahydrofurfurylpolyethylenglycol 200, Tri(ethylenglycol)divinylether, wasserfreies Triethylenglycol, Triethylenglycolmonoethylether.

Besonders bevorzugt ist der Waschpuffer ausgewählt aus der Gruppe enthaltend Tetraglykol, Tetraethylenglykol, 1,3-Butandiol, 1,2-Butandiol und Diethylenglycol monoethylether.

Des Weiteren ist Gegenstand der vorliegenden Erfindung ein Verfahren zur Extraktion von Nukleinsäuren aus einer Lösung umfassend die Schritte:
(a) Hinzufügen eines Bindungsmediators zu der Nukleinsäure enthaltenden Lösung
(b) in Kontakt bringen der Lösung enthaltend den Bindungsmediator und die Nukleinsäuren mit einer Oberfläche unter chaotropen und/oder Hoch-Salz-Bedingungen
(c) Bindung bzw. Adsorption der Nukleinsäuren an die Oberfläche,
(d) Waschen der Oberfläche gemäß dem erfindungsgemäßen Verfahren zum Waschen von an Oberflächen immobilisierten Nukleinsäuren
(e) Wiedergewinnung der Nukleinsäuren, welche an die Oberfläche gebunden oder adsorbiert sind, durch Elution.

Chaotrope Bedingungen werden durch die Zugabe von chaotropen Substanzen erreicht. Als chaotrop werden chemische Substanzen bezeichnet, die geordnete Wasserstoff-Brückenbindungen in wässrigen Lösungen auflösen. Sie vermindern damit den hydrophoben Effekt und wirken denaturierend auf Proteine, da die treibende Kraft der Proteinfaltung die Zusamrnenlagerung der hydrophoben Aminosäuren im Wasser ist. Beispiele für chaotrope Substanzen sind Bariumsalze, Guanidiniumhydrochlorid, Thiocyanate wie Guanidiniumthiocyanat, Perchlorate oder auch Natriumchlorid. Chaotrope Salze können gemäß ihres Löslichkeitsproduktes in Konzentrationsbereichen zwischen 1 M und 8 M eingesetzt werden.

Hoch-Salz Bedingungen bedeutet hochkonzentrierte Salzlösungen, wobei die Konzentration der Salze in der Lösung mindestens *1 M* beträgt, bevorzugt 1-*4M* beträgt.
Es können jedoch auch alternative Maßnahmen zu chaotropen oder Hoch-Salz-Bedingungen getroffen werden, die den gleichen Effekt erzielen, nämlich die Bindung der zu reinigenden Nukleinsäuren an die Oberfläche.

Bevorzugterweise wird der Bindungsmediator ausgewählt aus der Gruppe enthaltend Diethylenglycolmonoethylether, Diethylenglycolmonoethyletheracetat, Furfurylalkohol, Poly(1-vinylpyrrolidon-co-2-dimethylammoethylmethacrylat), Poly(2-ethyl-2-oxazolin), Poly(4-ammonium-styrol-sulfonsäure), Tetraethylenglycoldimethylether, Tetraetlylenglycol, Tetrahydrofurfurylpolyethylenglycol 200 und Triethylenglycolmonoethylether.

Der Fachmann kann auch durch Gemische der genannten Bindungsmediatoren erfolgreich Ethanol im Bindungsprozess bei der Nukleinsäurepräparation ersetzen. Da Ethanol-haltige Lösungen bis 24% (vol/vol) nicht als HAZMAT klassifiziert werden, können auch Gemische der Bindungsmediatoren mit Ethanol eingesetzt werden

Die Bindungsmediatoren liegen bevorzugt in folgenden Konzentrationen vor:
Diethylenglycolmonoethylether (DGME) [CAS 111-90-0] - Konzentrationsbereich 70-99 Vol%, bevorzugte Konzentration 99,0%; in Kombination mit Ethanol: 60-80 Vol% DGME und 16-24 Vol% Ethanol
Diethylenglycolmonoethyletheracetat (DGMEA) [CAS 112-15-] - Konzentrationsbereich 70-99Vol%, bevorzugte Konzentration 99,0Vol%; in Kombination mit Ethanol: 60-80Vol% DGMEA und 16-24Vol% Ethanol
Furfurylalkohol [CAS 98-00-] - Konzentrationsbereich 20-30Vol%, bevorzugte Konzentration 30Vol%
Poly(1-vinylpyrrolidon-co-2-dimethylaminoethylmethacrylat) [CAS 30581-59-0) - Konzentrationsbereich 3-5Vol%, bevorzugte Konzentration 5Vol%
Poly(2-ethyl-2-oxazolin) [CAS 25805-17-] - Konzentrationsbereich 9-15Vol% (w/v), bevorzugte Konzentration 12Vol%; in Kombination mit Ethanol: 22,5Vol% (w/v) und 16-24Vol% (v/v) Ethanol
Poly(4-ammoniumstyrolsulfonsäure) [CAS 29965-34-] - Konzentrationsbereich 8-22Vol% (w/v), bevorzugte Konzentration 12Vol%; in Kombination mit Ethanol: 8-22 (w/v) und 24Vol% (v/v) Ethanol
Tetraethylenglycoldimethylether [CAS 143-24-] - Konzentrationsbereich 70-98Vol%, bevorzugte Konzentration 98Vol%; in Kombination mit Ethanol: 73,5Vol% und 24Vol% Ethanol
Tetraglycol (Tetrahydrofurfurylpolyethylenglycolether)[CAS 9004-76-] - bevorzugte Konzentration mit Ethanol: 75Vol% und 16-24Vol% Ethanol
Tetrahydrofurfurylpolyethylenglycol 200 [CAS 31692-85-0] - Konzentrationsbereich 70-100Vol%, bevorzugte Konzentration 100Vol%
Triethylenglycolmonoethylether [CAS 112-50-5] - Konzentrationsbereich 70-90Vol%, bevorzugte Konzentration 90Vol%

Elutionspuffer sind in der Regel gepufferte Niedrigsalzlösungen mit neutralem bis leicht alkalischem pH Wert (zB: Puffer TE 10 mM Tris/Cl pH 8, 1 mM EDTA). Der Fachmann benutzt zum Teil auch destilliertes Wasser.

In einer besonders bevorzugten Variante des erfindungsgemäßen Verfahrens ist der Waschpuffer Tetraglykol in einer Konzentration von 45Vol% bis 80Vol%, vorzugsweise 52Vol% bis 72Vol%; Bei Anwesenheit von 2 bis 4 M, vorzugsweise 2,5 M an Chaotrop, wie z.B. Guanidinium Hydrochlorid liegt die bevorzugte Konzentration an Tetraglykol bei 45 bis 55 Vol%, vorzugsweise bei 50Vol%.

In einer anderen besonders bevorzugten Variante des erfindungsgemäßen Verfahrens ist der Waschpuffer Tetraethylenglykol in einer Konzentration von 45 bis 80Vol%, vorzugsweise von 50Vol% bis 70Vol%. Bei Anwesenheit von 2 bis 4 M, vorzugsweise 2,5 M an Chaotrop, wie z.B. Guanidinium Hydrochlorid liegt die bevorzugte Konzentration an Tetraethylenglykol bei 45 bis 55Vol%, vorzugsweise bei 50Vol%.

In einer anderen besonders bevorzugten Variante des erfindungsgemäßen Verfahrens ist der Waschpuffer 1,3-Butandiol in einer Konzentration von 45 bis 85Vol%, vorzugsweise von 51Vol% bis 80Vol%. Bei Anwesenheit von 2 bis 4 M, vorzugsweise 2,5 M an Chaotrop, wie z.B. Guanidinium Hydrochlorid liegt die bevorzugte Konzentration an 1,3-Butandiol bei 45 bis 55Vol%, vorzugsweise bei 49Vol%.

In einer anderen besonders bevorzugten Variante des erfindungsgemäßen Verfahrens ist der Waschpuffer 1,2-Butandiol in einer Konzentration von 41Vol% bis 71Vol%. Bei Anwesenheit von 2 bis 4 M, vorzugsweise von 2,5 M an Chaotrop, wie z.B. Guanidinium Hydrochlorid liegt die bevorzugte Konzentration an 1,2-Butandiol bei 41 bis 55Vol%, vorzugsweise bei 49Vol%.

In einer anderen besonders bevorzugten Variante des erfindungsgemäßen Verfahrens ist der Waschpuffer in einer Konzentration von 38 bis 98Vol%, vorzugsweise von 60Vol% bis 98Vol%. Bei Anwesenheit von 2 bis 4 M, vorzugsweise von 2,5 M an Chaotrop, wie z.B. Guanidinium Hydrochlorid liegt die bevorzugte Konzentration an 1,2-Butandiol bei 38 bis 45 Vol%, vorzugsweise bei 42Vol%.

Die Oberfläche, an welche die Nukleinsäuren adsorbiert werden, können auf Materialien basieren, die aus der folgenden Gruppe ausgewählt sein können: Silica-Materialien, carboxylierte Oberflächen, Zeolithe und Titaniumdioxid.

Bei dem erfindungsgemäßen Verfahren zur Extraktion von Nukleinsäuren aus einer Lösung werden die chaotropen und oder Hoch-Salz-Bedingungen des Reaktionsschrittes b) bevorzugt durch den Zusatz von chaotropen Salzen wie Kaliumiodid, Guanidinllydrochlorid, Guanidinthiocyanat oder Natriumchlorid zu der Nukleinsäure enthaltenden Lösung erreicht.

Die Nukleinsäure kann DNA wie beispielsweise genomische DNA sein. Die Nukleinsäure kann auch RNA wie beispielsweise Gesamt-RNA sein. Bei der Nukleinsäure kann es sich um einzelsträngige oder doppelsträngige Nukleinsäure, beispielsweise um kurze doppelsträngige DNA Fragmente, handeln.

Die Nukleinsäure-enthaltende Lösung kann durch einen Lysevorgang aus einem Nukleinsäure enthaltenden Material gewonnen werden.

Das Nukleinsäure enthaltende Material kann ausgewählt sein aus der Gruppe umfassend Blut, Gewebe, Abstrichpräparate, Bakterienkulturen, Urin, Zellsuspensionen und adherierende Zellen, PCR-Reaktionsgemische und *in vitro*-Nukleinsäuremodifikationsreaktionsgemische. Das Nukleinsäure enthaltenden Material kann menschliches, tierisches oder pflanzliches Material enthalten.

Die Nukleinsäure-enthaltende Lösung kann aus einer biochemischen Nukleinsäuremodifizierungsreaktion gewonnen sein.

Es ist bevorzugt, dass der Nukleinsäure enthaltenden Lösung Tenside zugesetzt werden. Diese Tenside werden bevorzugt in Konzentrationsbereichen von 0,1 Vol% bis 10 Vol% eingesetzt. Des Weiteren können eine Schaumbildung verhindernde Agentien (Anti-Foams) zugesetzt werden, bevorzugt in einem Bereich von 0,01 bis 1 Gew%.

Gegenstand der vorliegenden Erfindung ist ebenfalls ein Reagenzienkit zum Waschen von an Oberflächen immobilisierten Nukleinsäuren umfassend eine Lösung 1 umfassend einen Waschpuffer, wobei der Waschpuffer im Wesentlichen frei von Ethanol ist.

Gegenstand der vorliegenden Erfindung ist insbesondere ein Reagenzienkit zum Waschen von an Oberflächen immobilisierten Nukleinsäuren umfassend
- eine Lösung 1 umfassend den Waschpuffer ausgewählt aus der Gruppe enthaltend C3-und C4 Alkyldiole, sowie kurzkettige Ethylengylcol-Derivate und diverse wasserlösliche polymere Verbindungen und wobei der Waschpuffer im Wesentlichen frei von Ethanol ist.

In einer bevorzugten Ausführungsform des Reagenzienkits zum Waschen von an Oberflächen immobilisierten Nukleinsäuren umfasst der Reagenzienkit
- eine Lösung 1 umfassend den Waschpuffer ausgewählt aus der Gruppe enthaltend 1,2-Butandiol, 1,2-Propandiol, 1,3-Butandiol, 1-Methoxy-2-propanolacetat, 3-Methyl-1,3,5-pentantriol, DBE-2 dibasischer Ester, DBE-3 dibasischer Ester, DBE-4 dibasischer Ester, DBE-5 dibasischer Ester, DBE-6 dimethyladipat, Diethylenglycolmonoethylether (DGME), Diethylenglycolmonoethyletheracetat (DGMEA), Ethyllactat, Ethylenglykol, Poly(2-ethyl-2-oxazolin), Poly(4-styrolsulfonsäure-co-maleinsäure)-Natriumsalz-Lösung, Tetraethylenglycol (TEG), Tetraglycol (Tetrahydrofurfurylpolyethylenglycolether), Tetrahydrofurfurylpolyethylenglycol 200, Tri(ethylenglycol)divinylether, wasserfreies Triethylenglycol, Triethylenglycolmonoethylether.

In einer besonders bevorzugten Ausführungsform des Reagenzienkits zum Waschen von an Oberflächen immobilisierten Nukleinsäuren umfasst der Reagenzienkit
- eine Lösung 1 umfassend den Waschpuffer ausgewählt aus der Gruppe umfassend Tetraglykol, Tetraethylenglykol, 1,3-Butandiol, 1,2-Butandiol und Diethylenglycolmonoethylether.

Gegenstand der vorliegenden Erfindung ist auch ein Reagenzienkit zur Extraktion von Nukleinsäuren aus einer Lösung umfassend den vorstehend genannten Reagenzienkit und zusätzlich umfassend
- ein Gemisch 2 umfassend Bindungsmediator, und
- ein Gemisch 3 umfassend ein Elutionsmittel.

Bevorzugt wird der Bindungsmediator ausgewählt aus der Gruppe umfassend Diethylenglycolmonoethylether, Diethylenglycolmonoethyletheracetat, Furfurylalkohol, Poly(1-vinylpyrrolidon-co-2-dimethylaminoethylmethacrylat), Poly(2-ethyl-2-oxazolin), Poly(4-ammonium-styrol-sulfonsäure), Tetraethylenglycol-dimethylether, Tetraethylenglycol, Tetrahydrofurfuryl-polyethylenglycol 200 und Triethylenglycolmonoethylether.

Der Fachmann kann auch durch Gemische der genannten Bindungsmediatoren erfolgreich Ethanol in Bindungsprozess bei der Nukleinsäurepräparation ersetzen. Da Ethanol-haltige Lösungen bis 24% (vol/vol) nicht als HAZMAT klassifiziert werden können auch Gemische der Bindungsmediatoren mit Ethanol eingesetzt werden

Die Bindungsmediatoren liegen bevorzugt in folgenden Konzentrationen vor:
Diethylenglycolmonoethylether (DGME) [CAS 111-90-0] - Konzentrationsbereich 70-99Vol%, bevorzugte Konzentration 99,0Vol%; in Kombination mit Ethanol: 60-80Vol% DGME und 16-24Vol% Ethanol
Diethylenglycolmonoethyletheracetat (DGMEA) [CAS 112-15-] - Konzentrationsbereich 70-99Vol%, bevorzugte Konzentration 99,0Vol%; in Kombination mit Ethanol: 60-80Vol% DGMEA und 16-24Vol% Ethanol
Furfurylalcohol [CAS 98-00-] - Konzentrationsbereich 20-30Vol%, bevorzugte Konzentration 30Vol% Poly(1-vinylpyrrolidon-co-2-dimethylaminocthylmethacrylat) [CAS 30581-59-0) - Konzentrationsbereich 3-5Vol%, bevorzugte Konzentration 5Vol%
Poly(2-ethyl-2-oxazolin) [CAS 25805-17-] - Konzentrationsbereich 9-15Vol% (w/v), bevorzugte Konzentration 12Vol%; in Kombination mit Ethanol: 22,5Vol% (w/v) und 16-24Vol% (v/v) Ethanol
Poly(4-ammonium styrolsulfonsäure) [CAS 29965-34-] - Konzentrationsbereich 8-22Vol% (w/v), bevorzugte Konzentration 12Vol%; in Kombination mit Ethanol: 8-22 (w/v) und 24Vol% (v/v) Ethanol
Tetraethylenglycoldimethylether [CAS 143-24-] - Konzentrationsbereich 70-98Vol%, bevorzugte Konzentration 98Vol%; in Kombination mit Ethanol: 73,5Vol% und 24Vol% Ethanol
Tetraglycol [CAS 9004-76-] - bevorzugte Konzentration mit Ethanol: 75Vol% und 16-24Vol% Ethanol
Tetrahydrofurfurylpolyethylenglycol 200 [CAS 31692-85-0] - Konzentrationsbereich 70-100Vol%, bevorzugte Konzentration 100Vol%
Triethylenglycolmonoethylether [CAS 112-50-5] - Konzentrationsbereich 70-90Vol%, bevorzugte Konzentration 90Vol%

Elutionspuffer sind in der Regel gepufferte Niedrigsalzlösungen mit neutralem bis leicht alkalischem pH Wert (zB: Puffer TE der Fa. QIAGEN GmbH, Hilden). Der Fachmann benutzt zum Teil auch destilliertes Wasser.

Gegenstand der vorliegenden Erfindung ist des Weiteren ein Reagenzienkit zur Extraktion von Nukleinsäuren aus einer Lösung umfassend den vorstehend genannten Reagenzienkit und umfassend eine weitere Lösung 4 umfassend einen Lysepuffer und eine Protease.

Bei dem erfindungsgemäßen Verfahren zur Extraktion von Nukleinsäuren aus einer Lösung werden die chaotropen und/oder Hoch-Salz-Bedingungen des Reaktionsschrittes b) wie oben bereits dargelegt bevorzugt durch den Zusatz von chaotropen Salzen wie Kaliumiodid, Guanidinhydrochlorid, Guanidinthiocyanat oder Natriumchlorid zu der Nukleinsäure enthaltenden Lösung erreicht.
Daher ist Gegenstand der vorliegenden Erfindung auch ein erfindungsgemäßer Reagenzienkit zur Extraktion von Nukleinsäuren aus einer Lösung, wobei eine der Lösungen ein chaotropes Salz enthält oder Hoch-Salz-Bedingungen aufweist.

Bevorzugt wird das chaotrope Salz ausgewählt aus einer Gruppe enthaltend Kaliumiodid, Guanidinhydrochlorid, Guanidinthiocyanat und Natriumchlorid.

Gegenstand der vorliegenden Erfindung ist auch die Verwendung eines erfindungsgemäßen Reagenzienkits für die Extraktion von Nukleinsäuren aus biologischen Materialien wie Blut, Gewebe, Abstrichpräparate, Bakterien, Zellsuspensionen und adherierende Zellen.

Gegenstand der vorliegenden Erfindung ist auch die Verwendung eines erfindungsgemäßen Reagenzienkits für die Aufreinigung von Nukleinsäuren aus biochemischen Reaktionsgemischen, PCR-Reaktionsgemischen und *in vitro-*Nukleinsäuremodifikationsreaktionsgemischen.

Bei den in der vorliegenden Anmeldung beschriebenen Produkten, Puffern und Protokollen (Verfahrensanleitungen) handelt es sich um publizierte Dokumente und kommerziell erhältliche Produkte der Firma QIAGEN GmbH, Hilden, Deutschland, wenn nicht anders gekennzeichnet.

### Figurenbeschreibung

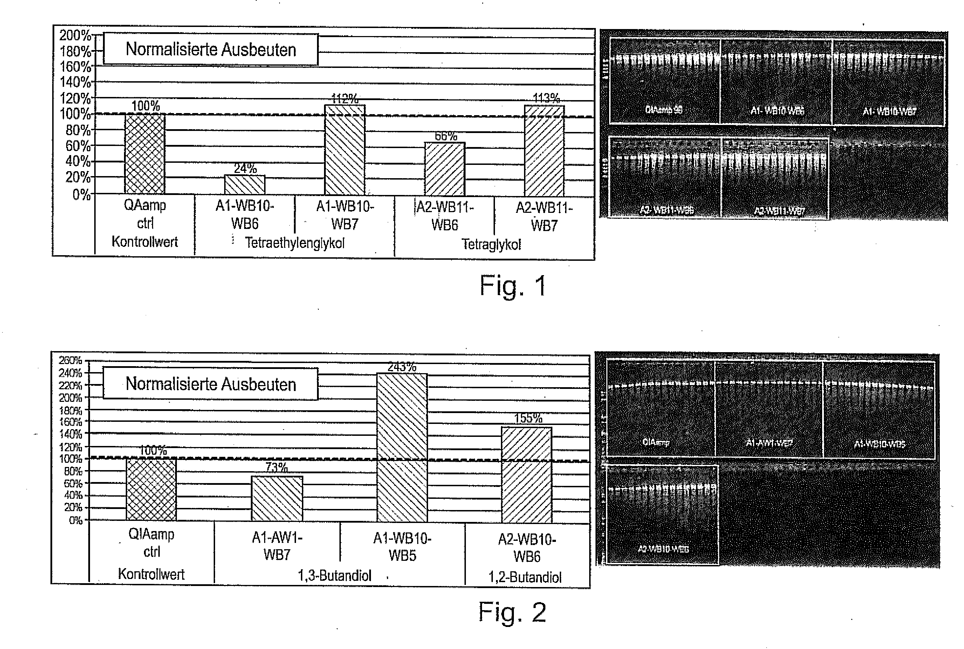

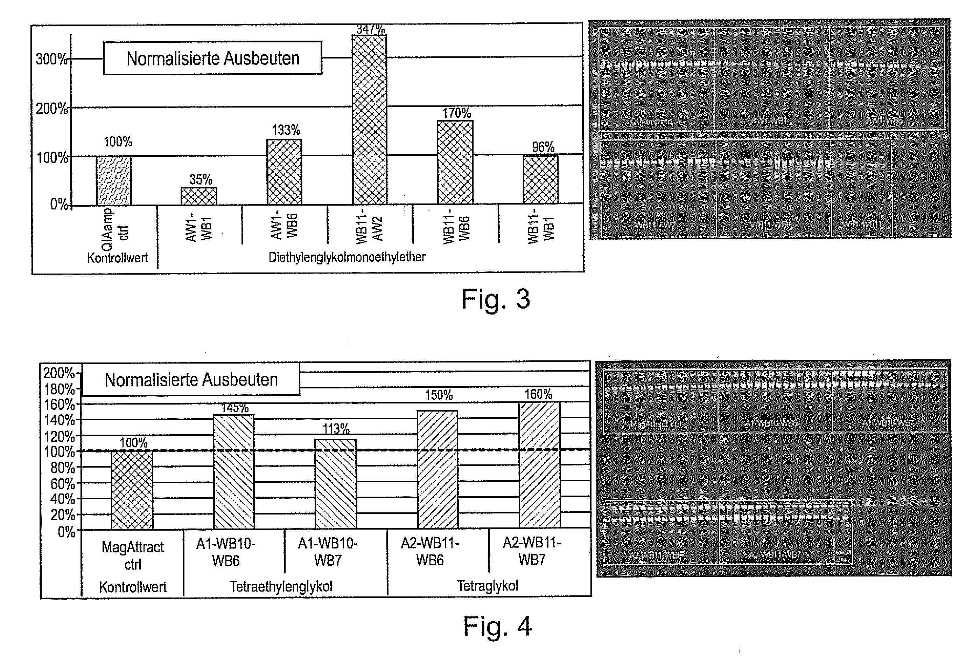

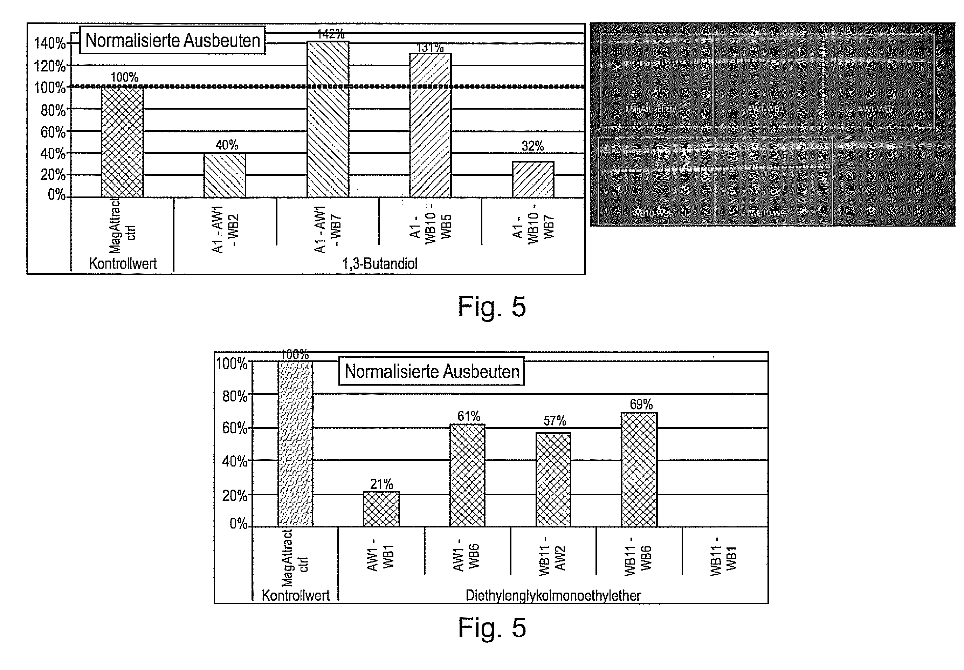

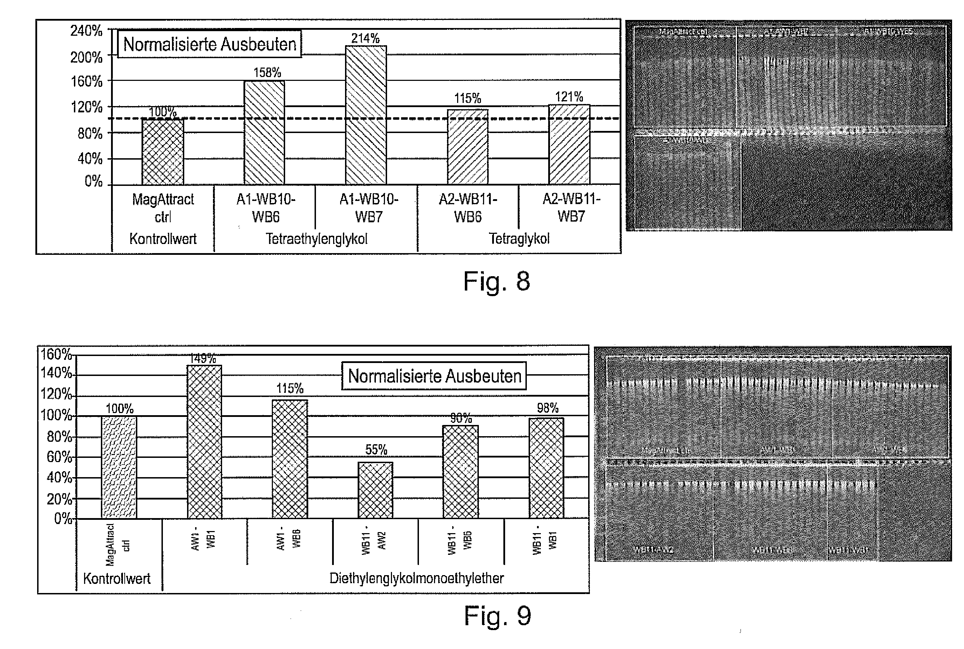

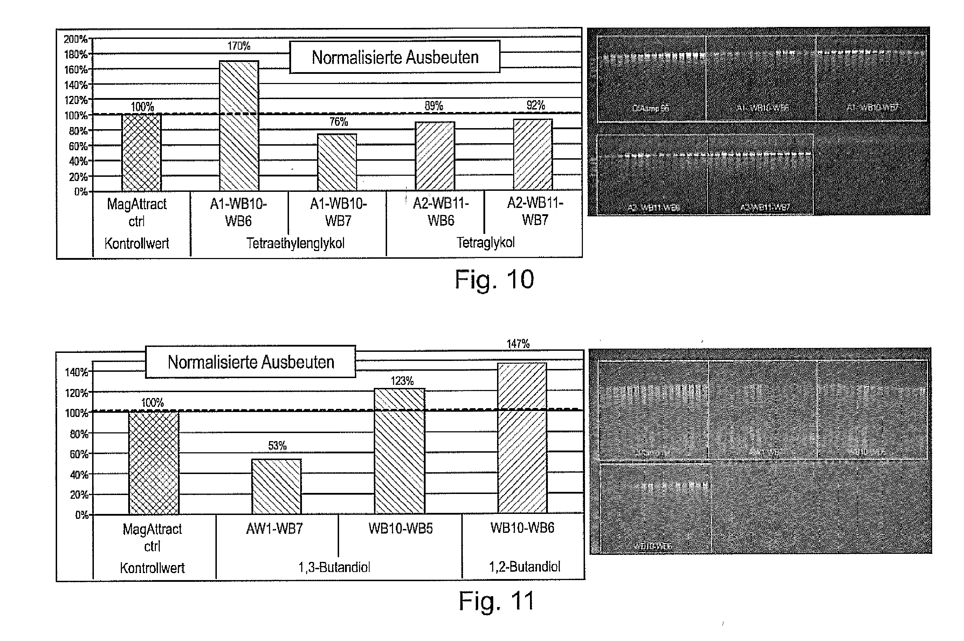

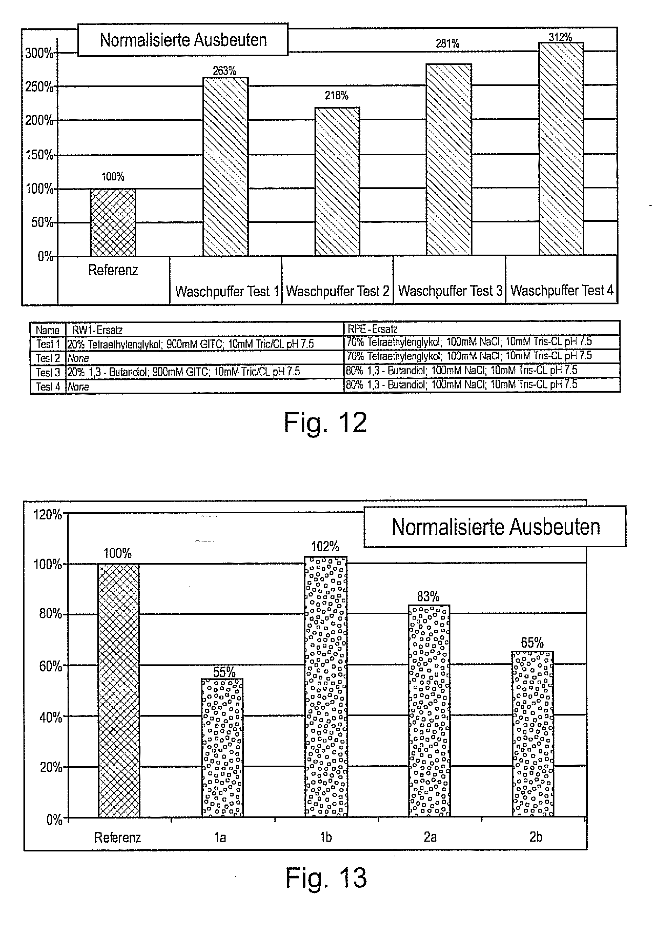

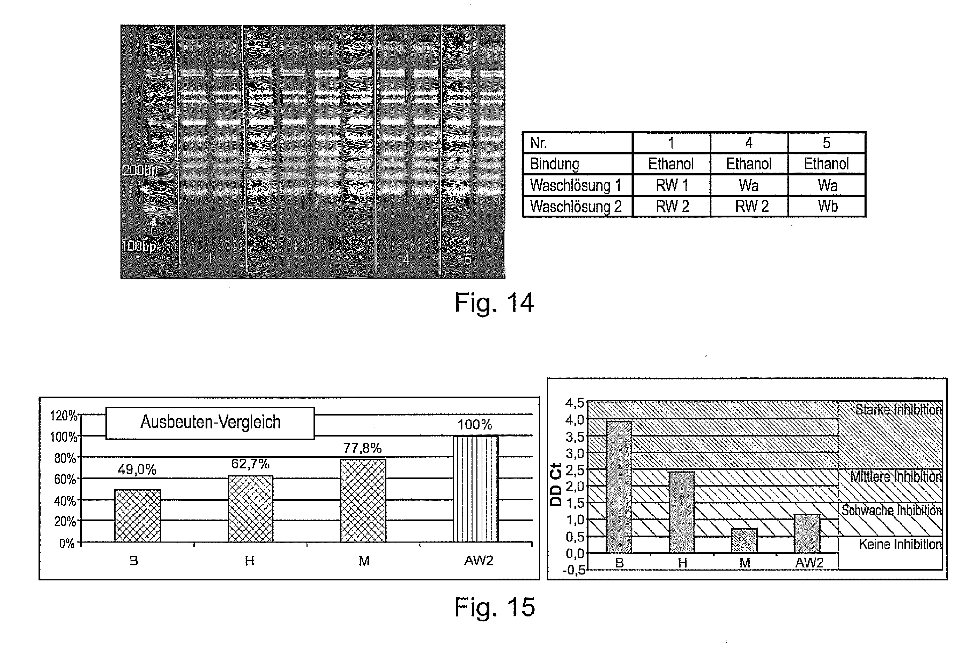

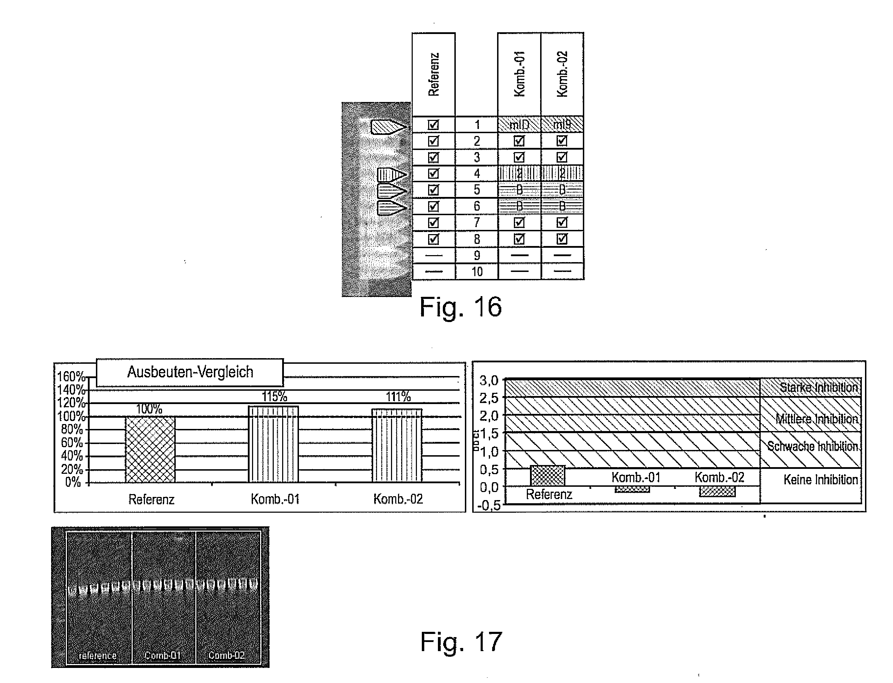

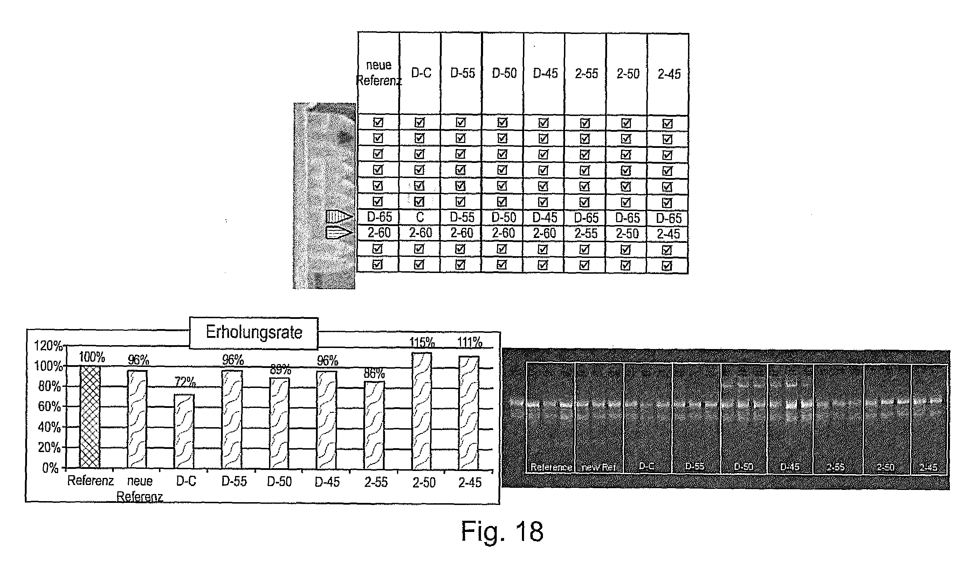

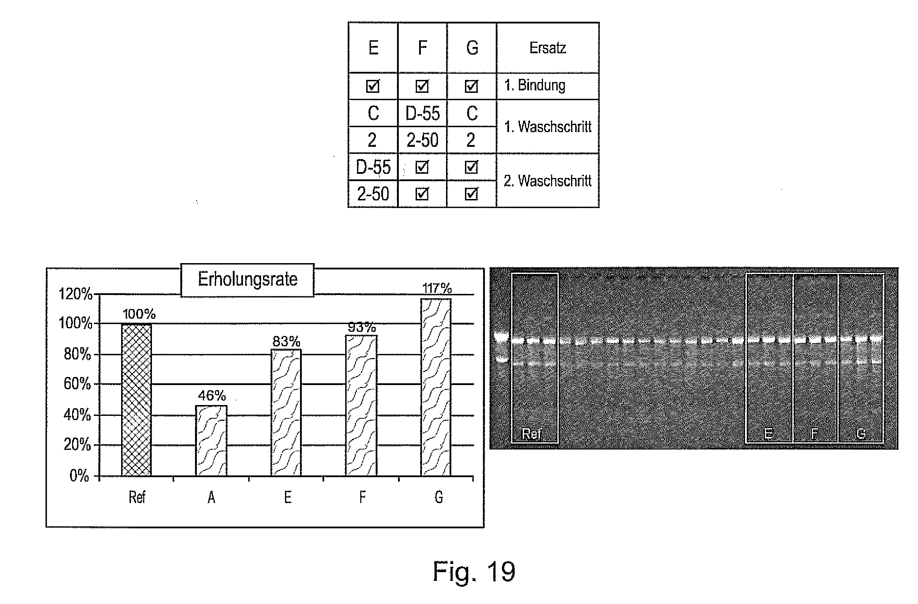

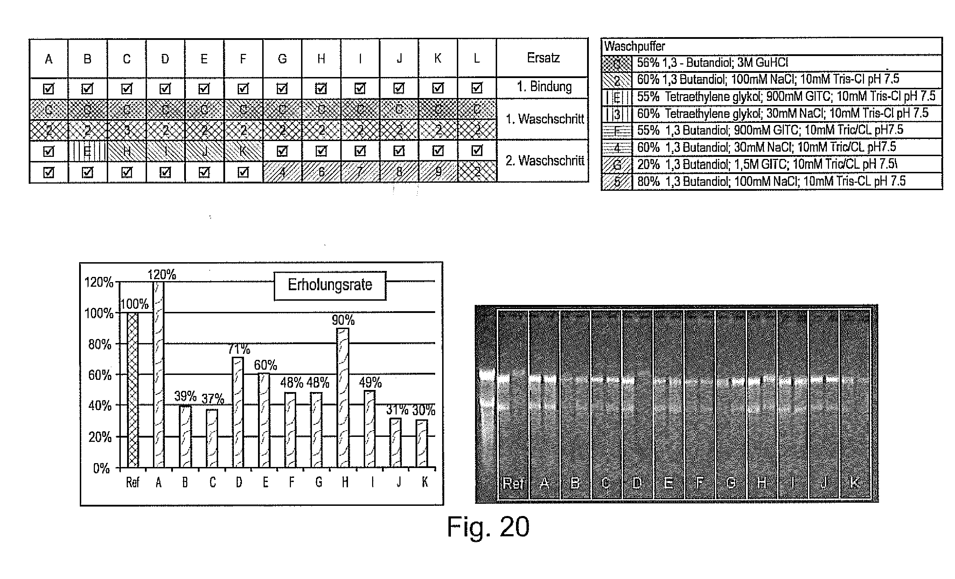

**Fig 1****:** Verhalten von Tetraethylenglycol und Tetraglycol, verwendet im QIAamp^{®} 96 Spin Blut Protokoll
   Links: Normalisierte Ausbeuten, ermittelt durch β-actin qPCR; rechts: Agarosegel mit den verschiedenen Proben
   A1: Tetraethylenglycol
   Erster Waschpuffer: WB10: 48,5Vol% Tetraethylenglycol; 2,5 M GuHCl
   Zweiter Waschpuffer:WB6: 69,8Vol% Tetraethylenglycol ; 100mM NaCl; 10mM Tris-Cl pH 7.5
   Zweiter Waschpuffer: WB7: 50,4Vol%Tetraethylenglycol ; 100mM NaCl; 10mM Tris-Cl pH 7.5
   A2: Tetraglycol
   Erster Waschpuffer: WB11: 50,0Vol% Tetraglycol ; 2,5 M GuHCl
   Zweiter Waschpuffer:WB6: 72,0Vol% Tetraglycol ; 100mM NaCl; 10mM Tris-Cl pH 7.5
   Zweiter Waschpuffer: WB7: 52,0Vol% Tetraglycol ; 100mM NaCl; 10mM Tris-Cl pH 7.5
**Fig 2****:** Verhalten von 1,3- Butandiol und 1,2- Butandiol, verwendet im QIAamp^{®} 96 Spin Blut-Protokoll
   Links: Normalisierte Ausbeuten, ermittelt durch β-actin qPCR; rechts: Agarosegel mit den verschiedenen Proben
   A1: 1,3-Butandiol
   Erster Waschpuffer: WB 10: 49,0Vol% 1,3- Butandiol ; 2,5 M GuHCl
   Zweiter Waschpuffer: WB5: 80,0Vol% 1,3 -Butandiol; 100mM NaCl; 10mM Tris-Cl pH 7.5
   Zweiter Waschpuffer: WB7: 51,0Vol% 1,3- Butandiol; 100mM NaCl, 10mM Tris-Cl pH 7.5
   A2: 1,2-Butandiol
   Erster Waschpuffer: WB10: 49,0Vol% 1,2-Butandiol; 2,5 M GuHCl
   Zweiter Waschpuffer: WB6: 71,0Vol% 1,2-Butandiol ; 100mM NaCl; 10mM Tris-Cl pH 7.5
**Fig 3****:** Verhalten von Diethylenglycolmonoethylether, verwendet im QIAamp^{®} 96 Spin Blut-Protokoll
   Links: Normalisierte Ausbeuten, ermittelt durch β-Actin qPCR; rechts: Agarosegel mit den verschiedenen Proben
   Erster Waschpuffer: QIAGEN Puffer AW1
   Erster Waschpuffer: WB11: 42Vol% Diethylenglycolmonoethylether; 2,5 M GuHCl
   Zweiter Waschpuffer: WB1. 98Vol% Diethylenglycolmonoethylether
   Zweiter Waschpuffer: WB6: 71Vol% Diethylenglycolmonoethylether; 100mM NaCl; 10mM Tris-Cl pH 7.5
**Fig 4****:** Verhalten von Tetraethylenglycol und Tetraglycol, verwendet im BioSprint^{®} 96 DNA Blut-Protokoll
   Links: Normalisiert Ausbeuten, ermittelt durch β-aActin qPCR; rechts: Agarosegel mit den verschiedenen Proben
   A1: Tetraethylenglycol
   Erster Waschpuffer: WB10: 48,5Vol% Tetraethylenglycol; 2,5 M GuHCl
   Zweiter Waschpuffer: WB6: 69,8Vol% Tetraethylenglycol; 100mM NaCl; 10mM Tris-Cl pH 7.5
   Zweiter Waschpuffer: WB7: 50,4Vol%Tetraethylenglycol ; 100mM NaCl; 10mM Tris-Cl pH 7.5
   A2: Tetraglycol
   Erster Waschpuffer: WB11: 50,0Vol% Tetraglycol ; 2,5 M GuHCl
   Zweiter Waschpuffer: WB6: 72,0Vol% Tetraglycol ; 100mM NaCl; 10mM Tris-Cl pH 7.5
   Zweiter Waschpuffer: WB7: 52,0Vol% Tetraglycol ; 100mM NaCl; 10mM Tris-Cl pH 7.5
**Fig 5****:** Verhalten von 1,3- Butandiol (A1), verwendet im BioSprint^{®} 96 DNA Blut-Protokoll
   Links: Normalisierte Ausbeuten, ermittelt durch β-actin qPCR; rechts: Agarosegel mit den verschiedenen Proben
   Erster Waschpuffer: WB10: 49Vol% 1,3 Butandiol ; 2.5M GuHCl
   Zweiter Waschpuffer: WB2: 74Vol% 1,3 Butandiol
   Zweiter Waschpuffer: WB5: 80Vol% 1,3 Butandiol ; 100mM NaCl; 10mM Tris-Cl pH 7.5
   Zweiter Waschpuffer: WB7: 51Vol% 1,3 Butandiol ; 100mM NaCl; 10mM Tris-Cl pH 7.5
**Fig5****:** Verhalten von Diethylenglykolmonoethylether, verwendet im BioSprint® 96 DNA Blut-Protokoll
   Links: Normalisierte Ausbeuten, ermittelt durch β-actin qPCR; rechts: Agarosegel mit den verschiedenen Proben
   Erster Waschpuffer: QIAGEN Puffer AW1
   Erster Waschpuffer: WB11: 42Vol% Diethylenglykolmonoethylether; 2,5 M GuHCl
   Zweiter Waschpuffer: WB1. 98Vol% Diethylenglykolmonoethylether
   Zweiter Waschpuffer: WB6: 71 Vol% Diethylenglykolmonoethylether; 100mM NaCl; 10mM Tris-CI pH 7.5
**Fig 6****:** QIAquick® Aufreinigung der Gel Pilot 1kb-Leiter. Dargestellt sind die normalisierten Ausbeuten. Die besten Waschpuffer als Ersatz für den kommerziell erhältlichen Puffer PE enthalten 48-64Vol% Tetraethylenglykol; 24Vol% Ethanol; 100 mM NaCl; 10 mM Tris pH 7,5
**Fig 7****:** Verhalten von 1,3- Butandiol und 1,2- Butandiol, verwendet im BioSprint^{®} 96 Tissue-Protokoll
   Links: Normalisierte Ausbeuten, ermittelt durch Mouse-GapDH qPCR; rechts: Agarosegel A1: 1,3-Butandiol
   Erster Waschpuffer: WB10: 49,0Vol% 1,3- Butandiol ; 2,5 M GuHCl
   Zweiter Waschpuffer: WB5: 80,0Vol% 1,3 -Butandiol ; 100mM NaCl; 10mM Tris-Cl pH 7.5
   Zweiter Waschpuffer: WB7: 51,0Vol% 1,3- Butandiol ; 100mM NaCl; 10mM Tris-Cl pH 7.5
   A2: 1,2-Butandiol
   Erster Waschpuffer: WB10: 49,0Vol% 1,2-Butandiol ; 2,5 M GuHCl
   Zweiter Waschpuffer: WB6: 71,0Vol% 1,2-Butandiol ; 100mM NaCl; 10mM Tris-Cl pH 7.5
**Fig 8****:** Verhalten von Tetraethylenglycol und Tetraglycol, verwendet im BioSprint^{®} 96 Tissue-Protokoll
   Links: Normalisierte Ausbeuten, ermittelt durch Mouse-GapDH qPCR; rechts: Agarosegel A1: Tetraethylenglycol
   Erster Waschpuffer: WB10: 48,5Vol% Tetraethylenglycol ; 2,5 M GuHCl
   Zweiter Waschpuffer: WB6: 69,8Vol% Tetraethylenglycol ; 100mM NaCl; 10mM Tris-Cl pH 7.5
   Zweiter Waschpuffer: WB7: 50,4Vol% Tetraethylenglycol ; 100mM NaCl; 10mM Tris-Cl pH 7.5
   A2: Tetraglycol
   Erster Waschpuffer: WB 11: 50,0Vol% Tetraglycol ; 2,5 M GuHCl
   Zweiter Waschpuffer: WB6: 72,0Vol% Tetraglycol ; 100mM NaCl; 10mM Tris-Cl pH 7.5
   Zweiter Waschpuffer: WB7: 52,0Vol% Tetraglycol ; 1 00mM NaCl; 10mM Tris-Cl pH 7.5
**Fig 9****:** Verhalten von Diethylenglycolmonoethylether, verwendet im BioSprint^{®} 96 Tissue-Protokoll
   Links: Normalisierte Ausbeuten, ermittelt durch Mouse-GapDH qPCR; rechts: Agarosegel
   Erster Waschpuffer: WB 11: 42Vol% Diethylenglycolmonoethylether; 2,5 M GuHCl
   Erster Waschpuffer: Puffer AW1
   Zweiter Waschpuffer: WB1: 98Vol% Diethylenglycolmonoethylether
   Zweiter Waschpuffer: WB6: 71Vol% Diethylenglycolmonoethylether; 100mM NaCl; 10mM Tris-Cl pH 7.5
**Fig 10****:** Verhalten von Tetraethylenglycol und Tetraglycol, verwendet im DNeasy^{®} 96 Tissue-Protokoll
   Links: Normalisierte Ausbeuten, ermittelt durch Mouse-GapDH qPCR; rechts: Agarosegel
   A1: Tetraethylenglycol
   Erster Waschpuffer: WB10: 48,5Vol% Tetraethylenglycol ; 2,5 M GuHCl
   Zweiter Waschpuffer: WB6: 69,8Vol% Tetraethylenglycol ; 100mM NaCl; 10mM Tris-Cl pH 7.5
   Zweiter Waschpuffer: WB7: 50,4Vol% Tetraethylenglycol ; 100mM NaCl; 10mM Tris-Cl pH 7.5
   A2: Tetraglycol
   Erster Waschpuffer: WB11: 50,0Vol% Tetraglycol ; 2,5 M GuHCl
   Zweiter Waschpuffer: WB6: 72,0Vol% Tetraglycol ; 100mM NaCl; 10mM Tris-Cl pH 7.5
   Zweiter Waschpuffer: WB7: 52,0Vol% Tetraglycol ; 100mM NaCl; 10mM Tris-Cl pH 7.5
**Fig 11****:** Verhalten von 1,3- Butandiol und 1,2- Butandiol, verwendet im BioSprint^{®} 96 Tissue-Protokoll
   Links: Normalisierte Ausbeuten, ermittelt durch Mouse-GapDH qPCR; rechts: Agarosegel A1: 1,3- Butandiol
   Erster Waschpuffer: WB10: 49,0Vol% 1,3- Butandiol ; 2,5 M GuHCl
   Zweiter Waschpuffer: WB5: 80,0Vol% 1,3 -Butandiol ; 100mM NaCl; 10mM Tris-Cl pH 7.5
   Zweiter Waschpuffer: WB7: 51,0Vol% 1,3- Butandiol ; 100mM NaCl; 10mM Tris-Cl pH 7.5
   A2: 1,2-Butandiol
   Erster Waschpuffer: WB10: 49,0Vol% 1,2-Butandiol; 2,5 M GuHCl
   Zweiter Waschpuffer: WB6: 71,0Vol% 1,2-Butandiol ; 100mM NaCl; 10mM Tris-CI pH 7.5
**Fig 12****:** Verhalten von Tetraethylenglycol und 1,3- Butandiol, verwendet im RNeasy^{®} 96-Protokoll Normalisierte Ausbeuten, ermittelt durch Lamin RT- qPCR; verwendet wurden"293"-Zellen
**Fig 13****:** Verhalten von verschiedenen Ethanol-ersetzenden Chemikalien, verwendet im RNeasy^{®} 96-Protokoll
   Links: Normalisierte Ausbeuten, ermittelt durch Mouse-GapDH qPCR; rechts: Agarosegel Bindungszusatz
   "1" 98Vol% TetraGlyme
   "2" 73,5Vol% TetraGlyme; 24Vol% Ethanol
   Waschkombination "a":
   Erster Waschpuffer: 20Vol% Tetraethylenglycol; 900 mM GTC; 10 mM Tris/CL pH 7.5;
   Zweiter Waschpuffer: 70Vol% Tetraethylenglycol ; 100mM NaCl; 10mM Tris-Cl pH 7.5 Waschkombination"b":
   Erster Waschpuffer: 20Vol% 1,3- Butandiol; 900 mM GTC; 10 mM Tris/CL pH 7.5
   Zweiter Waschpuffer: 80Vol% 1,3 Butandiol ; 100mM NaCl; 10mM Tris-Cl pH 7.5
**Fig 14****:** Fragmentgröße-Ausschluss-Experiment RNeasy® schließt kleine RNAs (5,8 S; tRNA; miRNA; ...) während der Aufreinigung aus. Die Grenzgröße sind circa 150 Basen. Dieses Experiment zeigt, dass der Größen-Ausschluss der getesteten Chemikalien vergleichbar mit Ethanol als Referenz ist.
   Wa: 20Vol% 1,3- Butandiol; 900 mM GTC; 10 mM Tris/CL pH 7.5
   Wb: 60Vol% 1,3 Butandiol ; 100mM NaCl; 10mM Tris-Cl pH 7.5
**Fig 15****:** Verhalten von verschiedenen Ethanol-ersetzenden Chemikalien, verwendet im EpiTect Aufreinigungsprotokoll.
   Links: Normalisierte Ausbeuten, ermittelt durch CFF1 qPCR; rechts: "Delta Delta Ct"-Analyse von unterschiedlichen Probenvolumina. Die Berechnung vergleicht die gemessenen Delta Ct-Werte mit den theoretischen Delta Ct-Werten, was einen numerischen Wert für den Grad der PCR-Inhibition ergibt.
   B 90Vol% Tetraethylenglycol; 10mM Tris-Cl pH 7.5
   H 72Vol% 1,3 Butandiol; 100mM NaCl; 10mM Tris-Cl pH 7.5
   M 60Vol% Diethylcnglycolrnonoethylether; 100mM NaCl; 10mM Tris-Cl pH 7.5
**Fig 16****:** Kartuschenanordnung
   Puffer ML
   mlD 4,5 M GTC; 50 mM NH4Cl; 45 mM Tris pH 7,5; 20 mM EDTA; 2.0Vol% Triton-X-100
   ml9 4,5 M GTC; 1,0 M NaCl; 50 mM NH4Cl; 45 mM Tris pH 7,5; 20 mM EDTA; 2.0Vol% Triton-X-100
   MW1 Ersatz-Puffer gemäß der vorliegenden Erfindung
   2 49Vol% 1,3- Butandiol ; 2.5 MGuHCl
   MW2 Ersatz-Puffer gemäß der vorliegenden Erfindung
   B 60Vol% 1,3 Butandiol ; 100mM NaCl; 10mM Tris-Cl pH 7.5
**Fig 17****:** Verhalten von verschiedenen Ethanol-ersetzenden Chemikalien, verwendet im EZ1^{®} DNA Blut 200µl Protokoll
   Links oben: Normalisierte Ausbeuten, ermittelt durch β-actin qPCR; rechts: "Delta Delta Ct"-Analyse von unterschiedlichen Probenvolumina. Die Berechnung vergleicht die gemessenen Delta Ct-Werte mit den theoretischen Delta Ct-Werten, was einen numerischen Wert für den Grad der PCR-Inhibition ergibt. Links unten: Agarosegel
**Fig 18****:** Verhalten von verschiedenen Ethanol-ersetzenden Chemikalien, verwendet im zweiten Waschschritt (nach dem DNase-Verdau) des EZ1^{®} - RNA Protokolls
   Links: Kartuschenanordnung; links unten: Normalisierte Ausbeuten, ermittelt durch MapK2 RT qPCR; rechts unten: Agarosegel
   MW 1 Ersatz-Puffer gemäß der vorliegenden Erfindung
   "C" 56Vol% 1,3- Butandiol; 3 M GuHCl
   "D-65"65Vol% 1,3- Butandiol; 1.75 M GuHCl
   "D-55"55Vol% 1,3- Butandiol; 1.75 M GuHCl
   "D-50"50Vol% 1,3- Butandiol; 1.75 M GuHCl
   "D-45"45Vol% 1,3- Butandiol; 1.75 M GuHCl
   Puffer RPE Ersatz-Puffer gemäß der vorliegenden Erfindung
   "2-60" 60Vol% 1,3 Butandiol ; 100mM NaCl; 10mM Tris-CI pH 7.5
   "2-55" 55Vol% 1,3 Butandiol ; 100mM NaCl; 10mM Tris-Cl pH 7.5
   "2-50" 50Vol% 1,3 Butandiol ; 100mM NaCl; 10mM Tris-Cl pH 7.5
   "2-45" 45Vol% 1,3 Butandiol ; 100mM NaCl; 10mM Tris-Cl pH 7.5
**Fig 19****:** Verhalten von verschiedenen Ethanol-ersetzenden Chemikalien, verwendet im ersten und zweiten Waschschritt des EZI^{®}-RNA Protokolls
   Oben: Kartuschenanordnung; links: Normalisierte Ausbeuten, ermittelt durch MapK2 RT qPCR; rechts unten: Agarosegel
      MW1 Ersatz-Puffer gemäß der vorliegenden Erfindung
      "C" 56Vol% 1,3- Butandiol; 3 M GuHCl
      "D-55"55Vol% 1,3- Butandiol; 1.75 M GuHCl
      Puffer RPE Ersatz-Puffer gemäß der vorliegenden Erfindung
      "2" 60Vol% 1,3 Butandiol ; 100mM NaCl; 10mM Tris-Cl pH 7.5
      "2-50" 50Vol% 1,3 Butandiol ; 100mM NaCl; 10mM Tris-Cl pH 7.5
**Fig 20****:** Verhalten von verschiedenen Ethanol-ersetzenden Chemikalien, verwendet im ersten und zweiten Waschschritt des EZ1^{®} - RNA Protokolls. Oben: Kartuschenanordnung; links: Normalisierte Ausbeuten, ermittelt durch MapK2 RT qPCR; rechts unten: Agarosegel

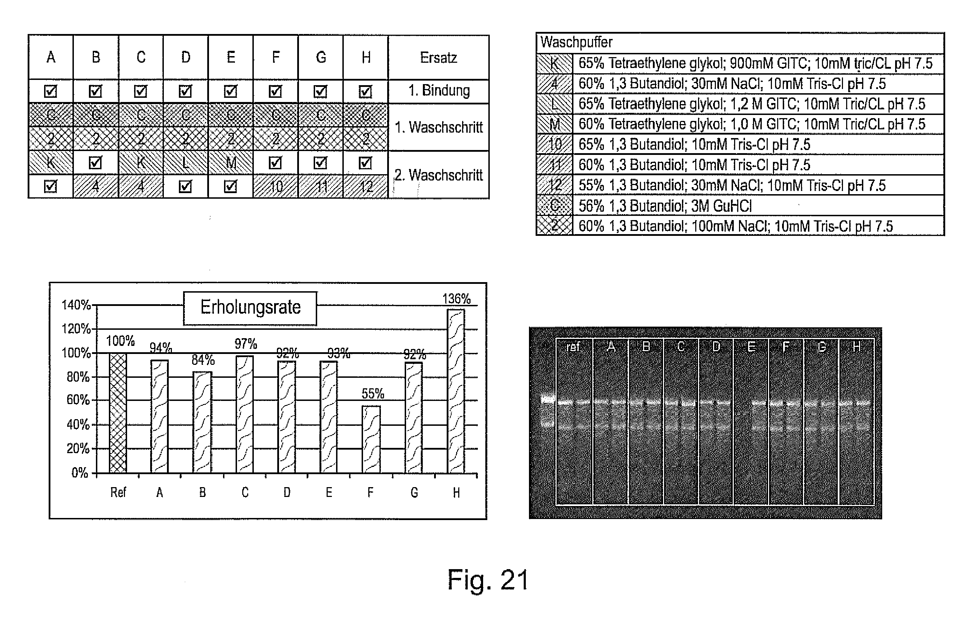

**Fig 21****:** Verhalten von verschiedenen Ethanol-ersetzenden Chemikalien, verwendet im ersten und zweiten Waschschritt des EZ1^{®} - RNA Protokolls. Oben: Kartuschenanordnung; links: Normalisierte Ausbeuten, ermittelt durch MapK2 RT qPCR; rechts unten: Agarosegel

Die folgenden Beispiele sollen die Erfindung näher erläutern:

### 1. Chemikalien und Testkits/Testprotokolle mit erfindungsgemäßen Waschpuffern

### 1.1 Chemikalien (zusammengefasste Aufstellung):

| **Name** | **CAS** |
|---|---|
| Tetraglycol, flüssig, rein | 9004-76-6 |
| Tetraethylenglycol purum, 97Vol% | 112-60-7 |
| 1,3-Butandiol purum, 98Vol% | 107-88-0 |
| 1,2-Butandiol purum, 98Vol% | 584-03-2 |
| Diethylenglycolmonoethyle ther, 99Vol% | 111-90-0 |

### 1.2 Testkits/Testprotokolle

### 1.2.1 "BioSprint® 96 DNA Blut"

BioSprint^{®} 96 mit Protokoll-Datei: "BS96_DNA_Blut_200"

Lyse
- 200 µl Blut
- 200 µl Puffer AL
- 20 µl QIAGEN Protease
- 15 min bei 56°C und 1400 rpm auf einem Thermomixer inkubieren

Bindung
- 200 µl Isopropanol zugeben
- 30 µl MagAttract^{®} Suspension G (QIAGEN GmbH) zugeben

Waschlösungen
Referenzprotokoll
   - 1x Puffer AW1 (650 µl)
   - 1x Puffer AW1 (500 µl)
   - 2x Puffer AW2 (500 µl)
      Puffer AW1 Ersatz-Puffer gemäß der vorliegenden Erfindung
      - 50Vol% Tetraglykol ; 2.5M GuHCl
      - 48,5Vol% Tetaethylenglycol ; 2.5M GuHCl
      - 49Vol% 1,3-Butandiol ; 2.5M GuHCl
      - 49Vol% 1,2-Butandiol ; 2.5M GuHCl
      - 42Vol% Diethylenglycolmonoethylether ; 2.5M GuHCl
      Puffer AW2 Ersatz-Puffer gemäß der vorliegenden Erfindung
      - 52Vol% Tetraglycol ; 100mM NaCl, 10mM Tris-Cl pH 7.5
      - 69,8Vol% Tetraethylenglycol ; 100mM NaCl; 10mM Tris-Cl pH 7.5
      - 80Vol% 1,3-Butandiol; 100mM NaCl; 10mM Tris-Cl pH 7.5
      - 71Vol% 1,2-Butandiol; 100mM NaCl; 10mM Tris-Cl pH 7.5
      - 71Vol% Diethylenglycolmonoethylether ; 100mM NaCl; 10mM Tris-Cl pH 7.5
Wasserspülung: 0,02Vol% Tween 20
Elution: 200 µl µl RNase-freies Wasser in MicroTubePacl-MicroPlate

### 1.2.2 QIAamp^{®} 96 Spin Blut-Protokoll

Lyse
- 200 µl Blut
- 200 µl Puffer AL
- 20 µl QIAGEN Protease
- Inkubation 15 min bei 56°C
   Bindung
- 200 µl Ethanol zugeben
- in S-Block mischen und in QIAamp^{®} 96-Platte transferieren
   Waschlösungen

Referenzverfahren
   ○ 1x Puffer AW1 (550 µl)
   ○ 1x Puffer AW2 (500 µl)
      Puffer AW1-Ersatzlösungen gemäß der vorliegenden Erfindung
      - 49Vol% 1,3-Butandiol ; 2.5M GuHCl
      - 49Vol% 1,2-Butandiol ; 2.5M GuHCl
      - 42Vol% Diethylenglycolmonoethylether; 2.5M GuHCl
      - 50Vol% Tetraglycol ; 2.5M GuHCl
      - 48,5Vol% Tetraethylenglycol ; 2.5M GuHCl
         Puffer AW2-Ersatzlösungen gemäß der vorliegenden Erfindung
      - 80Vol% 1,3-Butandiol; 100mM NaCl; 10mM Tris-Cl pH 7.5
      - 71Vol% 1,2-Butandiol; 100mM NaCl; 10mM Tris-CI pH 7.5
      - 71Vol% Diethylenglycolmonoethylether ; 100mM NaCl; 10mM Tris-Cl pH 7.6
      - 52Vol% Tetraglycol ; 100mM NaCl; 10mM Tris-Cl pH 7.5
      - 50,4Vol% Tetraethylenglycol ; 100mM NaCl; 10mM Tris-Cl pH 7.5

Elution: 200 µl µl RNase-freies Wasser im Elutions-Microtube Rack

### 1.2.3 "BioSprint^{®} 96 DNA Tissue"

BioSprint^{®}-96 Protokoll-Datei: "BS96_DNA_Blut_200"
Lyse
- 200 µl Lysat ( 25 mg Gewebe + 180 µl Puffer ATL + 20µl Proteinase K, über Nacht Inkubation 56°C)
- + 200 µl Puffer AE
   Bindung
- 200 µl Isopropanol zugeben
- + 30 µl MagAttract^{®} Suspension G
   Waschlösungen
   ○ 1x Puffer AW1 (650 µl)
   ○ 1x Puffer AW1 (500 µl)
   ○ 2x Puffer AW2 (500 µl)
Puffer AW1-Ersatzlösungen gemäß der vorliegenden Erfindung
- 48,5Vol% Tetraethylenglycol ; 2.5M GuHCl
- 50Vol% 1,2-Butandiol ; 2.5M GuHCl
- 50Vol% Tetraglycol ; 2.5M GuHCl
- 49Vol% 1,3-Butandiol ; 2.5M GuHCl
- 42Vol% Diethylenglycolmonoethylether; 2.5M GuHCl
Puffer AW2-Ersatzlösungen gemäß der vorliegenden Erfindung
- 50,4Vol% Tetraethylenglycol; 100mM NaCl; 10mM Tris-Cl pH 7.5
- 52Vol% 1,2-Butandiol ; 100mM NaCl; 10mM Tris-Cl pH 7.5
- 52Vol% Tetraglycol ; 100mM NaCl; 10mM Tris-CI pH 7.5
- 80Vol% 1,3-Butandiol; 100mM NaCl; 10mM Tris-Cl pH 7.5
- 98Vol% Diethylenglycolmonoethylether, reine Lösung
Wasserspülung: 0,02Vol% Tween® 20 (500 µl)
Elution: 200 µl RNase-freies Wasser in Microtube-Platte

### 1.2.4 "DNeasy^{®} 96 Tissue"

Lyse
- 200 µl Lysat ( 25mg Gewebe + 180 µl Puffer ATL + 20µl Proteinase K, Über Nacht inkubieren 56°C)
- + 200 µl Puffer AE
Bindung
- 200 µl Ethanol zugeben
- im S-Block mischen und in QIAamp^{®} 96-Platte transferieren
Waschlösungen
- 1x Puffer AW1 (650 µl)
- 1x Puffer AW2 (500 µl)
   Puffer AW1-Ersatzlösungen gemäß der vorliegenden Erfindung
- 48,5Vol% Tetraethylenglycol ; 2.5M GuHCl
- 49Vol% 1,2-Butandiol ; 2.5M GuHCl
- 49Vol% 1,3-Butandiol ; 2.5M GuHCl
- 50Vol% Tetraglycol ; 2.5M GuHCl
   Puffer AW2-Ersatzläsungen gemäß der vorliegenden Erfindung
- 69,8Vol% Tetraethylenglycol ; 100mM NaCl; 10mM Tris-Cl pH 7.5
- 71Vol% 1,2-Butandiol ; 100mM NaCl; 10mM Tris-Cl pH 7.5
- 80Vol% 1,3-Butandiol ; 100mM NaCl; 10mM Tris-Cl pH 7.5
- 52Vol% Tetraglycol ; 100mM NaCl; 10mM Tris-Cl pH 7.5
Elution: 200 µl RNase-freies Wasser in Elution Micro Tubes

### 1.2.5 RNeasy^{®} 96

Bindung
- 350 µl Puffer RLT - Lysat ("293" Zellen; 2x10⁵ Zellen/Probe)
- 350 µl Ethanol zugeben
- im S-Bloclc mischen und in RNeasy^{®} 96-Platte transferieren
Waschlösungen
- 2x Puffer RW1 (650 µl)
- 2x Puffer RPE (500 µl)
   Puffer RW1-Ersatzlösungen gemäß der vorliegenden Erfindung
- 20Vol% 1,3- Butandiol; 900 mM GTC; 10 mM Tris/CL pH 7.5
- 20Vol% Tetraethylenglycol; 900 mM GTC; 10 mM Tris/CL pH 7.5 Puffer RPE-Ersatzlösungen gemäß der vorliegenden Erfindung
- 80Vol% 1,3 Butandiol; 100mM NaCl; 10mM Tris-Cl pH 7.5
- 60Vol% Tetraethylenglycol; 100mM NaCl; 10mM Tris-CI pH 7.5
   Elution: 100 µl RNase-freies Wasser in Elution Micro Tubes

### 1.2.6 QIAquick^{®}

Bindung
- 1 Volumenprobe
- 5 Volumen Puffer PM zugeben (Standard-Referenzprotokoll)
- auf QIAamp^{®} MinElute Säule laden; 1' @ 8000rpm
   Waschlösung
- 1x Waschlösung : A; 1' @ 8000rpm
   Puffer PM-Ersatzlösung gemäß der vorliegenden Erfindung
- 64Vol% Tetraethylenglycol; 24Vol% Ethanol; 100 mM NaCl; 10 mM Tris pH 7,5
- trocken zentrifugieren
- Eluieren 40µl RNase-freies Wasser; 1' @ 8000rpm

### 1.2.7 Kein Gefahrgut - modifiziertes EpiTect^{®} Bisulfit-Protokoll

1) Bisulfit-DNA-Konversion nach Handbuch **EpiTeet^{®} Bisulfit-Kit der Fa QIAGEN GmbH**
2) Aufreinigung von Bisulfit-konvertierter DNA *Schritt* #:
   6. Die vollständigen Bisulfit-Reaktionen in saubere 1.5 ml-Mikrozentrifugenröhrchen transferieren. Fällungsmittel in der Lösung beeinflussen nicht die Leistung oder Ausbeute der Reaktion.
   7. 560 µl frisch präparierten Puffer BL, enthaltend 10 µg/ml Träger-RNA, zugeben. Die Lösung durch Vortexen mischen.
      Anmerkung: Träger-RNA ist nicht erforderlich bei Verwendung von >100 ng DNA.
   8. Die gesamte Mischung in die EpiTect® Schleudersäule transferieren.
   9. Die Säule bei Maximalgeschwindigkeit 1 min lang zentrifugieren. Durchfluss verwerfen und Schleudersäule wieder in das Sammelgefäß stellen.
   10. 500 µl Puffer BW (Waschpuffer) in die Schleudersäule geben und bei Maximalgeschwindigkeit 1 min zentrifugieren. Durchfluss verwerfen und Schleudersäule wieder in das Sammelgefäß stellen.
   11. 500 µl Puffer BD (Desulfonierungspuffer) in die Schleudersäule geben und 15 min bei Raumtemperatur inkubieren. Wenn Präzipitate im Puffer BD vorhanden sind, vermeiden Sie es, diese in die Schleudersäule zu transferieren. Anmerkung: Es ist wichtig, den Säulendeckel vor der Inkubation zu schließen.
   12. Die Säule bei Maximalgeschwindigkeit 1 min zentrifugieren. Den Durchfluss verwerfen und die Schleudersäule zurück in das Sammelgefäß stellen.
   13. 500 µl Puffer BW zugeben und bei Maximalgeschwindigkeit 1 min zentrifugieren. Den Durchfluss verwerfen und die Schleudersäule zurück in das Sammelgefäß stellen.
   14. Schritt 13 einmal wiederholen.
   15. Die Schleudersäule in ein neues 2 ml-Sammelgefäß stellen und die Schleudersäule bei Maximalgeschwindigkeit 1 min zentrifugieren, um verbleibende Flüssigkeitsreste zu entfernen. Anmerkung: Wenn die aufgereinigte DNA für Verwendung in einer Echtzeit-PCR bestimmt ist, die Zentrifugierzeit auf 5 min verlängern.
   16. Die Schleudersäule in ein sauberes 1.5 ml-Mikrozentrifugierröhrchen stellen. 20 µl Puffer EB auf die Mitte der Membran geben. Die aufgereinigte DNA durch Zentrifugation für 1 min bei circa 15,000 x g (12,000 rpm) eluieren.

Puffer BW-Ersatzlösungen gemäß der vorliegenden Erfindung für Protokollschritt 13
- 60Vol% Diethylenglycolmonoethylether; 100mM NaCl; 10mM Tris-Cl pH 7.5
- 72Vol% 1,3 Butandiol ; 100mM NaCl; 10mM Tris-Cl pH 7.5

### 1.2.8 EZ1^{®} DNA Blut 200ul-Protokoll

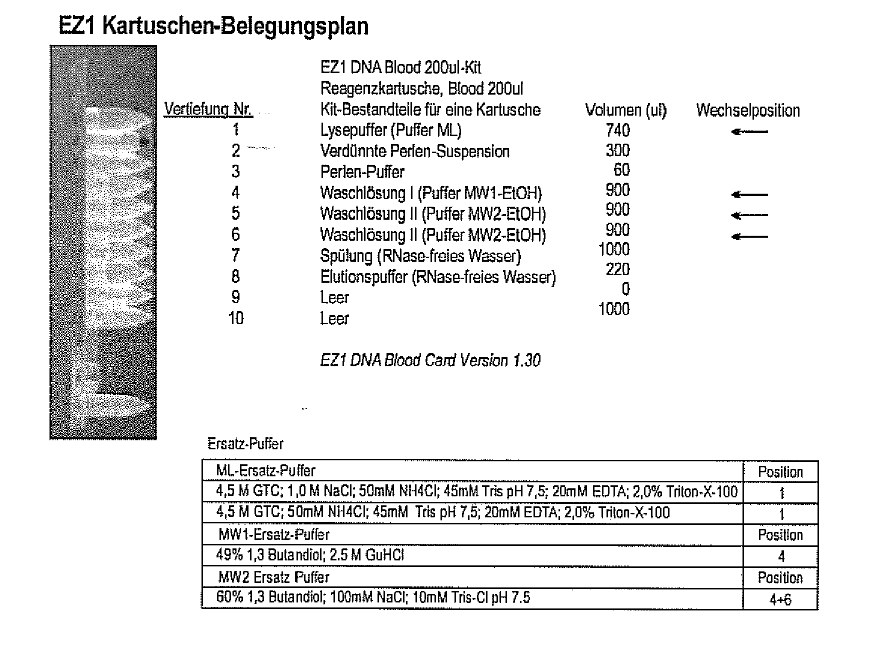

### 1.2.9 EZ1^{®} - RNA-Protokoll

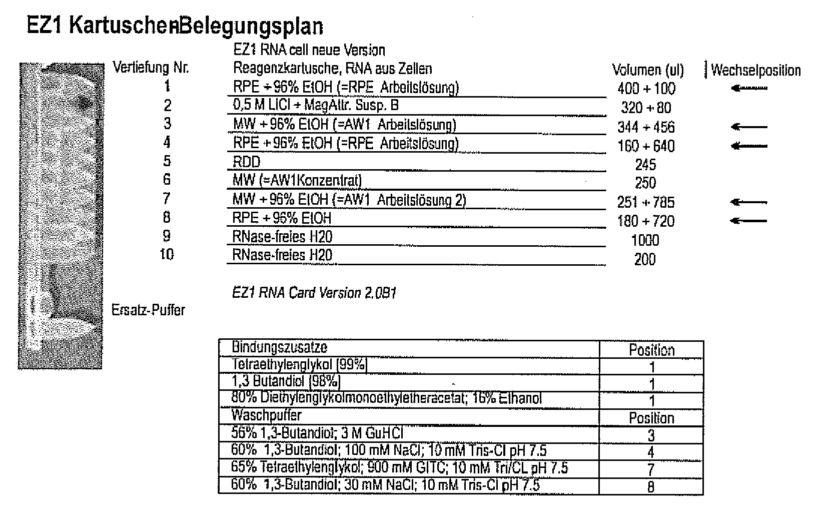
EZ1 KartuschenBelegungsplan
EZ1 RNA cell neue Version
Vertiefung Nr. | Reagenzkartusche, RNA aus Zellen | Volumen (ul) | Wechselposition
1 | RPE + 96% EtOH (=RPE Arbeitslösung) | 400 + 100 | ←
2 | 0,5 M LiCl + MagAttr. Susp. B | 320 + 80 |
3 | MW + 96% EtOH (=AW1 Arbeitslösung) | 344 + 456 | ←
4 | RPE + 96% EtOH (=RPE Arbeitslösung) | 160 + 640 | ←
5 | RDD | 245 |
6 | MW (=AW1Konzentrat) | 250 |
7 | MW + 96% EtOH (=AW1 Arbeitslösung 2) | 251 + 785 | ←
8 | RPE + 96% EtOH | 180 + 720 | ←
9 | RNase-freies H20 | 1000 |
10 | RNase-freies H20 | 200 |
EZ1 RNA Card Version 2.0B1
Ersatz-Puffer

Bindungszusatze | Position
Tetraethylenglykol (99%) | 1
1,3 Butandiol (98%) | 1
80% Diethylenglykolmonoethyletheracetat; 16% Ethanol | 1
Waschpuffer | Position
56% 1,3-Butandiol; 3 M GuHCl | 3
60% 1,3-Butandiol; 100 mM NaCl; 10 mM Tris-Cl pH 7.5 | 4
65% Tetraethylenglykol; 900 mM GITC; 10 mM Tri/CL pH 7.5 | 7
60% 1,3-Butandiol; 30 mM NaCl; 10 mM Tris-Cl pH 7.5 | 8

### 2. Ergebnisbeispiele

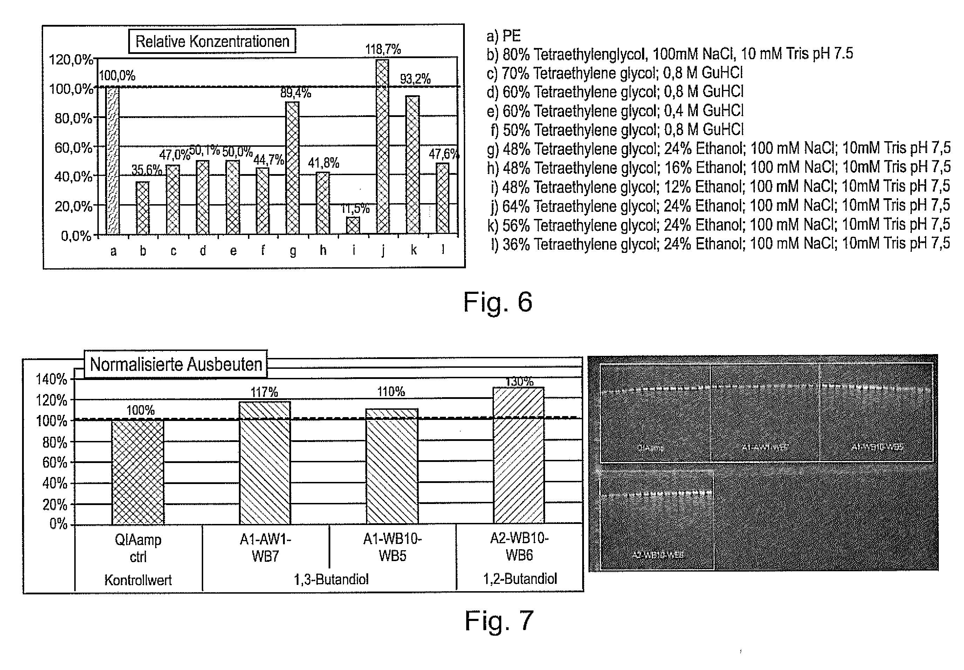

Die Ergebnisse der erfindungsgemäßen Testkits/Testprotokolle werden in den Figuren 1-21 dargestellt.

Die hier dargestellten Untersuchungen konzentrierten sich auf die Ethanol-ersetzenden Chemikalien in Waschpuffern für die Silica-vermittelte Präparation von Nukleinsäuren. Das getestete Probenmaterial reichte von Blut bis zu Geweben sowie Nukleinsäure-Modifizierungsreaktions-Aufreinigungsprodukten. Zusätzlich wurde die Aufreinigung verschiedener Typen von Nukleinsäuren getestet (genomische DNA; Total-RNA, kleine doppelsträngige DNA-Fragmente). Die identifizierten Chemikalien wurden mit Verfahren getestet, bei denen Säulen mit Silica-basierten Membranen oder Silica-umhüllte Magnetpartikel verwendet wurden.

### Chemikalie

### CAS

### Konzentration / Pufferzusammensetzungen der in den Beispielen eingesetzten Ersatzlösungen gemäß der vorliegenden Erfindung

1,2- Butanediol [1,2-Butandiol]
   584-03-2
   49Vol% 1,2-Butandiol ; 2.5M GuHCl
   71Vol% 1,2-Butandiol; 100mM NaCl; 10mM Tris-Cl pH 7.5
1,2-Propandiol
   57-55-6
   99Vol% 1,2-Propandiol
   74Vol% 1,2-Propandiol, 100mM NaCl; 10mM Tris-Cl pH 7.5
1,3- Butanediol [1,3-Butandiol]
   107-88-0
   49Vol% 1,3-Butandiol ; 2.5M GuHCl
   56Vol% 1,2-Butandiol; 3 M GuHCl
   20Vol% 1,3- Butandiol; 900 mM GTC; 10 mM Tris/CL pH 7.5
   72 - 80Vol% 1,3-Butandiol; 30 - 100mM NaCl; 10mM Tris-Cl pH 7.5
1-Methoxy-2-propanol acetate [2-Methoxy-1-methylethylacetat]
   108-65-6
   100Vol% 1-Methoxy-2-propanolacetat
3-Methyl-1,3,5-pentantriol
   7564-64-9
   80 - 40Vol% 3-Methyl-1,3,5-pentantriol
   60Vol% 3-Methyl-1,3,5-pentantriol, 100mM NaCl; 10mM Tris-Cl pH 7.5
DBE-2 dibasic ester [dibasischer Ester]
   MFCD00191969
   100 - 50 Vol% DBE-2
DBE-3 dibasic ester [dibasischer Ester]
   MFCD00191969
   100 - 50 Vol% DBE-3
DBE-4 dibasic ester [dibasischer Ester; Dimethylsuccinat]
   106-65-0
   100 - 50 Vol% DBE-4
DBE-5 dibasic ester [dibasischer Ester; Dimethylglutarat]
   1119-40-0
   100 - 50 Vol% DBE-5
DBE-6 dimethyl adipate [dibasischer Ester; Dimethyladipat]
   627-93-0
   100 - 50 Vol% DBE-5
Diethylene glycol monoethyl ether (DGME) [Diethylenglycolmonoethylether, Ethyldiglykol] 111-90-0
   42Vol% DGME ; 2.5M GuHCl
   71Vol% DGME ; 100mM NaCl; 10mM Tris-Cl pH 7.5
Diethylenglycolmonoethyletheracetat (DGMEA) [Ethyldiglykolacetat]
   112-15-2
   99,0Vol% DGMEA
   50-80Vol% DGMEA ; 100mM NaCl; 10mM Tris-Cl pH 7.5
Ethyl lactate [Ethyllactat]
   97-64-3
   100Vol% Ethyllactat
   75Vol% Ethyllactat, 100mM NaCl; 10mM Tris-Cl pH 7.5
Ethylene glycol [Ethylenglycol]
   107-21-1
   100Vol% Ethylenglycol
Poly(2-ethyl-2-oxazoline) [2-Ethyl-4,5-dihydro-1,3-oxazol]
   25805-17-8
   23Vol% Poly(2-ethyl-2-oxazoline), 100mM NaCl; 10mM Tris-Cl pH 7.5
Poly(4-styrolsulfonsäure-co-maleinsäure)
   68037-40-1
   25Vol% Poly(4-styrolsulfonsäure-co-maleinsäure)
   19Vol%Poly(4-styrolsulfonsäure-co-maleinsäure)
   100mM NaCl; 10mM Tris-Cl pH 7.5
Tetraethylene glycol (TEG) [Tetraethylenglycol]
   112-60-7
   48,5Vol% TEG ; 2.5M GuHCl
   20 - 65Vol% TEG; 900 mM GTC; 10 mM Tris/Cl pH 7.5
   50,4 - 60Vol% TEG ; 100mM NaCl; 10mM Tris-Cl pH 7.5
   64Vol% TEG, 24Vol% Ethanol;
   50Vol% TEG, 50Vol% Poly(propylenglycol), MW ∼725 (CAS 25322-69-4)
Tetraglycol
   9004-76-6
   50Vol% Tetraglycol; 2.5M GuHCl
   52Vol% Tetraglycol ; 100mM NaCl; 10mM Tris-Cl pH 7.5
Tenahydrofurfurylpolyethylenglycol 200
   31692-85-0
   100 Vol% (purum)
   75Vol% Tetrahydrofurfuryl PEG 200, 100mM NaCl; 10mM Tris-Cl pH 7.5
Tri(ethylene glycol) divinyl ether [Triethylenglycoldivinylether]
   765-12-8
   75 - 98Vol% Tri(ethyleneglycol)divinylether
Triethylene glycol anhydrous [Triethylenglycol, wasserfrei]
   112-27-6
   97Vol% Triethylenglycol, wasserfrei,
   75Vol% Triethylenglycol, wasserfrei, 100mM NaCl; 10mM Tris-Cl pH 7.5
Triethylene glycol monoethyl ether [Triethylenglycolmonoethylether]
   112-50-5
   90Vol% Triethylenglycolmonoethylether,
   68Vol% Triethylenglycolmonoethylether, 100mM NaCl; 10mM Tris-Cl pH 7.5

**Tabelle 1 kommerziell erhältliche Produkte der Fa. QIAGEN, im Rahmen der Beispiele eingesetzt**

| MagAttract^{®} suspension G | Suspension mit Magnetpartikeln |
|---|---|
| Puffer PE | Waschpuffer mit schwacher organischer Base |
| Puffer AE | Niedrigsalz-Puffer |
| Puffer EB | Wässriger Elutionspuffer |
| Puffer TE | Elutionspuffer; 10mM TrisCL, mM EDTH pH8 |
| RNase-freies Wasser | Reinstwasser |
| Puffer AL | Lysepuffer enthaltend Guanidiniumhydrochlorid |
| Puffer RLT | Puffer umfassend Thiocyanat |
| Puffer ATL | Puffer enthaltend EDTA und SDS |
| Puffer ML | Puffer enthaltend Guanidiniumthiocyanat und t-Octylphenoxypolyoxyethanol |
| Puffer AP1 | Puffer enthaltend EDTA und SDS |
| Puffer AW1 | Waschpuffer enthaltend Guanidiniumhydrochlorid |
| Puffer AW2 | Waschpuffer enthaltend Natriumazid |
| Puffer RW1 | Alkoholhaltiger Puffer mit Guanidiniumsalz |
| Puffer RPE | Wässriger Puffer |
| MTP-MP | Mikrotiterplatte |
| EMT | Elutionsmikrotubes |
| Puffer PM | Bindepuffer enthaltend Guanidiniumchlorid und 2-Propanol |
| Puffer MW1 (enthält | Einsatzpuffer enthaltend Guanidiniumhydrochlorid und |
| Ethanol) | Ethanol |
| Puffer MW2 (enthält Ethanol) | Puffer mit Lithiumchlorid und Ethanol |
| GTC | Guanidiniumthiocyanat |
| MW1 Ersatz-Puffer (enthält kein Ethanol) | Einsatzpuffer enthaltend Guanidiniumhydrochlorid |
| MW2 Ersatz-Puffer (enthält kein Ethanol) | Puffer mit Lithiumchlorid |
| RDD | RNAse-freier Puffer |
| AlAamp Spin | Puffer mit Lithiumchlorid und Ethanol |
| K-AC | Kaliumacetat |
| EGME | Ethylenglycolmonomethyl ether |
| MagSep | Magnetische Separation |
| MagStep | Schritt zur Magnetischen Separation |
| Puffer BL | Guanidiniumthiocyanat enthaltender Puffer |
| Puffer BW | Waschpuffer zur effektiven Entsalzung von DNA |
| Puffer BD | NaOH enthaltender Puffer |

## Patentansprüche

1. Verfahren zum Waschen von an Oberflächen immobilisierten Nukleinsäuren, wobei die Nukleinsäuren mit einem Waschpuffer in Kontakt gebracht werden, welcher weniger als 24 Vol % Alkohol mit 1-3 C-Atomen und mindestens ein weiteres Lösungsmittel enthält, welches ausgewählt ist aus Alkandiolen und -triolen mit 2-6 C-Atomen, Monocarbonsäureestern und Dicarbonsäurediestern mit 2-6 C-Atomen im Säureteil und 1-4 C-Atomen im Alkoholteil; (Poly)Ethylenglykole und Ether- und Esterderivate davon, wobei "poly" 2-200 repetitive Ethylenglykol-Einheiten bezeichnet; Poly-(2-Ethyl-2-oxazolin); Poly(4-Styrolsulfonsäure-co-Maleinsäure) Natriumsalzlösung.

2. Verfahren zum Waschen von an Oberflächen immobilisierten Nukleinsäuren gemäß Anspruch 1 oder 2, wobei der Waschpuffer Lösungsmittel enthält welche ausgewählt sind aus der Gruppe enthaltend 1,2-Butandiol, 1,2-Propandiol, 1,3-Butandiol, 1-Methoxy-2-propanolacetat, 3-Methyl-1,3,5-pentantriol, DBE-2, DBE-3, DBE-4, DBE-5, DBE-6, Diethylenglykolmonoethylether (DGME), Diethylenglykolmonoethyletheracetat (DGMEA), Ethyllactat, Ethylenglykol, Poly(2-ethyl-2-oxazolin), Poly(4-styrolsulfonsäure-co-Maleinsäure) Natriumsalzlösung, Tetraethylenglykol (TEG), Tetraglykol, Tetrahydrofurfurylpolyethylenglykol 200. Tri(ethylenglykol)divinyl-ether, Triethylenglykol wasserfrei, Triethylenglykolmonoethylether.

3. Verfahren zum Waschen von an Oberflächen immobilisierten Nukleinsäuren gemäß einem der Ansprüche 1 bis 3, wobei der Waschpuffer Lösungsmittel enthält welche ausgewählt sind aus der Gruppe enthaltend Tetraglykol, Tertraethylenglykol, 1,3-Butandiol, 1,2-Butandiol und Triethylenglykolmonoethylether.

4. Verfahren zur Extraktion von Nukleinsäuren aus einer Lösung umfassend die Schritte:
(a) Hinzufügen eines Bindungsmediators zu der Nukleinsäure enthaltenden Lösung
(b) in Kontakt bringen der Lösung den Bindungsmediator und die Nukleinsäuren enthaltenden Lösung mit einer Oberfläche unter chaotropen und/oder Hoch-Salz-Bedingungen
(c) Bindung bzw. Adsorption der Nukleinsäuren an eine Oberfläche,
(d) Waschen der Oberfläche gemäß einem Verfahren nach einem der Ansprüche 1 bis 4.
(e) Wiedergewinnung der Nukleinsäuren, welche an die Oberfläche adsorbiert sind, durch Elution.

5. Verfahren gemäß einem der Ansprüche 1-4, wobei die Nukleinsäure genomische DNA ist.

6. Verfahren gemäß einem der Ansprüche 1-4, wobei die Nukleinsäure Gesamt-RNA ist,

7. Verfahren gemäß einem der Ansprüche 1-4, wobei die Nukleinsäuren kurze doppelsträngige DNA Fragmente sind.

8. Reagenzienkit zum Waschen von an Oberflächen immobilisierten Nukleinsäuren umfassend
- eine Lösung 1 umfassend einen Waschpuffer, wobei der Waschpuffer wie in Anspruch 1-4 definiert ist.

9. Reagenzienkit gemäß Anspruch 8 zusätzlich umfassend
- eine Lösung 2 umfassend Bindungsmediator, und
- eine Lösung 3 umfassend ein Elutionsmittel.

10. Reagenzienkit gemäß Anspruch 9, umfassend eine weitere Lösung 4 umfassend einen Lysepuffer und eine Protease.

11. Verwendung eines Reagenzienkits gemäß einem der Ansprüche 8 bis 10 für die Extraktion von Nukleinsäuren aus biologischen Materialien wie Blut, Gewebe, Abstrichpräparaten, Bakterien, Zellsuspensionen und adherierenden Zellen.

12. Verwendung gemäß Anspruch 11 für die Aufreinigung von Nukleinsäuren aus biochemischen Reaktionen, PCR-Reaktionen und *in vitro-*Nukleinsäuremodifikationsreaktionen.
